# EUROPEAN PATENT APPLICATION

(11) **EP 3 301 187 A1**
(43) Date of publication of application: **04.04.2018**
(21) Application number: 17170740.9
(22) Date of filing: 12.05.2017
(51) Int. Cl.: C12Q 1/68, G06F 19/22

(54) **NON-INVASIVE FETAL SEX DETERMINATION SYSTEM AND METHOD THEREOF**

(30) Priority: 30.09.2016 TW 105131733
(71) Applicant: Yourgene Bioscience, New Taipei City 238 (TW)
(72) Inventor: CHAN, Chia-Han, 242 New Taipei City (TW)
(74) Representative: Lang, Christian

(57) **Abstract**

A non-invasive fetal sex determination system includes a sequencing platform and a non-transitory machine readable medium. The sequencing platform is for cleaving a DNA of a tested sample into a plurality of divided fragments, and the non-transitory machine readable medium is for storing a program used to detect the sexuality. The non-transitory machine readable medium includes an analyzing-processing module, a reference database and a comparing module. The analyzing-processing module is for cleaving a known Y chromosome sequence of a human body into a plurality of comparison fragments and comparing the divided fragments with the comparison fragments. The reference database is constructed by the analyzing-processing module, wherein the reference database includes a male area and a female area. The comparing module is for comparing the number with the reference database to determine the number falling within which area.

## Description

### BACKGROUND

### Technical Field

The present disclosure relates to a fetal sex determination system and method thereof. More particularly, the present disclosure relates to a non-invasion fetal sex determination system and method thereof.

### Description of Related Art

A pregnant woman will accept series of examinations to confirm the health status of the fetus during the pregnancy. In order to quest for the accuracy of the examined results, invasion examinations such as the chorionic villus sampling (CVS) and amniocentesis which examine the cells separated from the fetus are mostly used. However, those invasive examinations are dangerous not only to the pregnant woman but also to the fetus.

Research results indicate that the blood of the pregnant woman contains the DNA fragments of the fetus, so the current examinations tend to examine the blood of the pregnant woman to detect the related disease so as to reduce the dangers to the pregnant woman and the fetus. However, there are still some diseases are unable or difficult to detect, especially the diseases relate to the sex abnormality such as Y chromosome microdeletion.

Therefore, how to efficiently detect the diseases of the sex abnormality has become the major aim of the people in related business.

### SUMMARY

According to one aspect of the present disclosure, a non-invasive fetal sex determination system includes a sequencing platform and a non-transitory machine readable medium, wherein the sequencing platform is for cleaving a DNA of a tested sample into a plurality of divided fragments, and the non-transitory machine readable medium is for storing a program which is used to detect the sexuality of the DNA of the tested sample. The non-transitory machine readable medium includes an analyzing-processing module, a reference database and a comparing module. The analyzing-processing module is for cleaving a known Y chromosome sequence of a human body into a plurality of comparison fragments and for comparing the divided fragments with the comparison fragments so as to obtain a number of the comparison fragments which includes at least one of the divided fragments. The reference database is constructed by the analyzing-processing module analyzed after cleaving a plurality of reference biological samples by the sequencing platform, wherein the reference database includes a male area and a female area. The comparing module is for comparing the number with the reference database so as to determine that the DNA of the tested sample is male when the number falls within the male area and the DNA of the tested sample is female when the number falls within the female area.

According to the non-invasive fetal sex determination system of the foregoing aspect, wherein a length of the divided fragment ranges from 10 base pairs to 300 base pairs.

According to the non-invasive fetal sex determination system of the foregoing aspect, the analyzing-processing module includes a processing unit and a mathematical unit. The processing unit is for selecting the divided fragments with the length greater than a particular value and for cleaving the divided fragments selected based on the particular value into a plurality of analyzed fragments. The mathematical unit is for calculating the number of the comparison fragments which includes at least one of the analyzed fragments. The reference biological samples of the reference database are processed by the analyzing-processing module beforehand to construct the male area and the female area corresponding to the particular value, and the comparing module determines the DNA of the tested sample is male when the number falls within the male area and the DNA of the tested sample is female when the number falls within the female area.

According to the non-invasive fetal sex determination system of the foregoing aspect, the Y chromosome sequence of the human body is cleaved at the length of 50 kilo base pairs, the length of 100 kilo base pairs, or the length of 200 kilo base pairs.

According to the non-invasive fetal sex determination system of the foregoing aspect, the particular value is 51 base pairs, 101 base pairs, or 151 base pairs.

According to the non-invasive fetal sex determination system of the foregoing aspect, wherein the reference database further includes an abnormal area and the comparing module compares the number with the reference database so as to determine the number falls within the abnormal area, the male area, or the female area.

According to the non-invasive fetal sex determination system of the foregoing aspect, the reference biological samples of the reference database are processed by the analyzing-processing module beforehand to construct the male area, the female area, and the abnormal area corresponding to the particular value.

According to another aspect of the present disclosure, a non-invasive fetal sex determination method includes providing a sequencing procedure, providing an analyzing-processing procedure, acquiring a reference database, and providing a comparing procedure. The sequencing procedure is for cleaving a DNA of a tested sample into a plurality of divided fragments by a sequencing platform. The analyzing-processing procedure is for cleaving a known Y chromosome sequence of a human body into a plurality of comparison fragments and for comparing the divided fragments with the comparison fragments so as to obtain a number of the comparison fragments which includes at least one of the divided fragments. The reference database is constructed by an analyzing-processing module analyzed after cleaving a plurality of reference biological samples by the sequencing platform, wherein the reference database includes a male area and a female area. The comparing procedure is for comparing the number with the reference database so as to determine that the DNA of the tested sample is male when the number falls within the male area and the DNA of the tested sample is female when the number falls within the female area.

According to the non-invasive fetal sex determination method of the foregoing aspect, wherein a length of the divided fragment ranges from 10 base pairs to 300 base pairs.

According to the non-invasive fetal sex determination method of the foregoing aspect, the analyzing-processing module includes selecting the divided fragments with a length greater than a particular value and cleaving the divided fragments selected based on the particular value into a plurality of analyzed fragments, and calculating the number of the comparison fragments which includes at least one of the analyzed fragments. The reference biological samples of the reference database are processed by the analyzing-processing module beforehand to construct the male area and the female area corresponds to the particular value, and the comparing module determines the DNA of the tested sample is male when the number falls within the male area and the DNA of the tested sample is female when the number falls within the female area.

According to the non-invasive fetal sex determination method of the foregoing aspect, wherein the Y chromosome sequence of the human body is cleaved at the length of 50 kilo base pairs, the length of 100 kilo base pairs, or the length of 200 kilo base pairs.

According to the non-invasive fetal sex determination method of the foregoing aspect, wherein the particular value is 51 base pairs, 101 base pairs, or 151 base pairs.

According to the non-invasive fetal sex determination method of the foregoing aspect, wherein the reference database further includes an abnormal area and the comparing procedure is for comparing the number with the reference database to determine the number falling within the abnormal area, the male area, or the female area.

According to the non-invasive fetal sex determination method of the foregoing aspect, the reference biological samples of the reference database are processed by the analyzing-processing module beforehand to construct the male area, the female area, and the abnormal area corresponding to the particular value.

### BRIEF DESCRIPTION OF THE DRAWINGS

The present disclosure can be more fully understood by reading the following detailed description of the embodiment, with reference made to the accompanying drawings as follows:
Fig. 1 is a block diagram of a non-invasive fetal sex determination system according to one embodiment of the present disclosure;
Fig. 2 is a chart of a reference database of the non-invasive fetal sex determination system of Fig. 1;
Fig. 3 is another chart of the reference database of the non-invasive fetal sex determination system of Fig. 1;
Fig. 4 is still another chart of the reference database of the non-invasive fetal sex determination system of Fig. 1;
Fig. 5 is a flow chart of a non-invasive fetal sex determination method according to another embodiment of the present disclosure;
Fig. 6 is a block diagram of a non-invasive fetal sex determination system according to still another embodiment of the present disclosure;
Fig. 7 is a chart of a reference database of the non-invasive fetal sex determination system of Fig. 6;
Fig. 8 is another chart of the reference database of the non-invasive fetal sex determination system of Fig. 6;
Fig. 9 is a flow chart of a non-invasive fetal sex determination method according to yet another embodiment of the present disclosure; and
Fig. 10 is a result chart of a conventional non-invasive fetal sex determination system.

### DETAILED DESCRIPTION

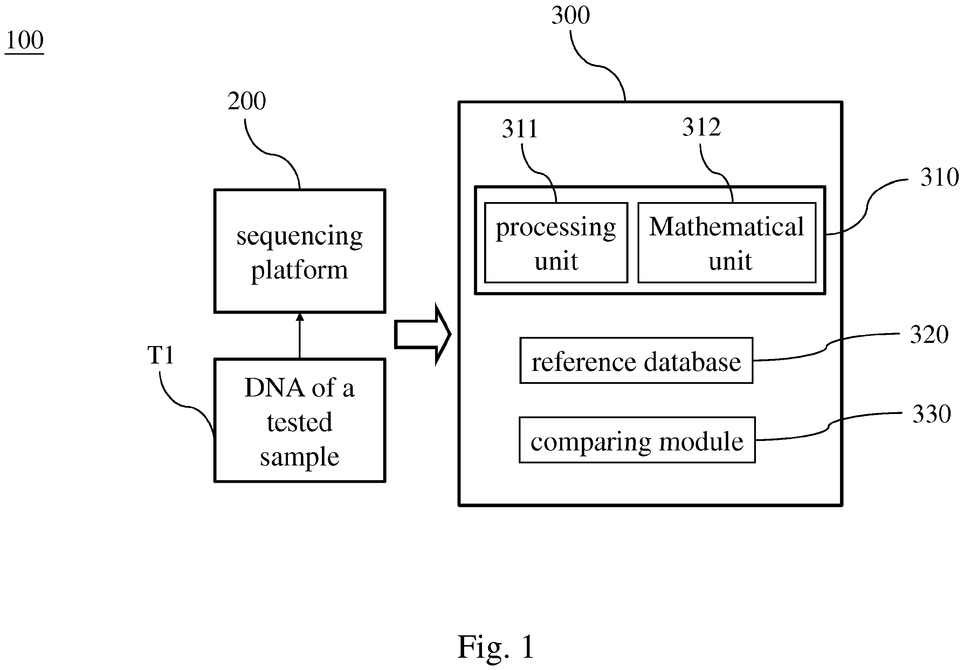

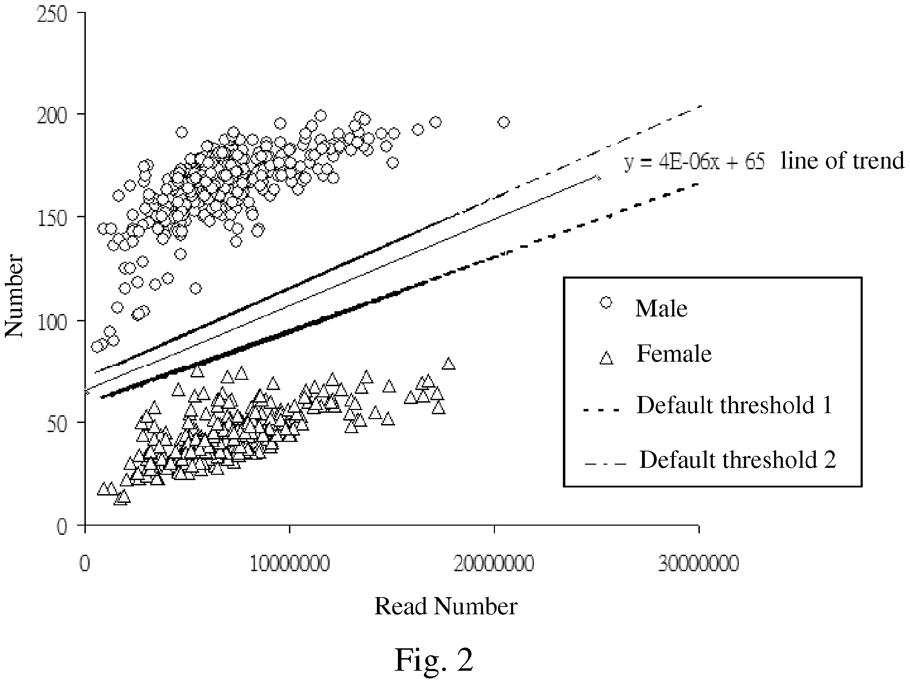

Fig. 1 is a block diagram of a non-invasive fetal sex determination system 100 according to one embodiment of the present disclosure, and Fig. 2 is a chart of a reference database 320 of the non-invasive fetal sex determination system 100 of Fig. 1. The non-invasive fetal sex determination system 100 includes a sequencing platform 200 and a non-transitory machine 300, wherein the sequencing platform 200 is for cleaving a DNA of a tested sample T1 into a plurality of divided fragments, and the non-transitory machine readable medium 300 is for storing a program used to detect the sexuality of the DNA of the tested sample T1. The non-transitory machine 300 includes an analyzing-processing module 310, a reference database 320 and a comparing module 330. The analyzing-processing module 310 is for cleaving a known Y chromosome sequence of a human body into a plurality of comparison fragments and comparing the divided fragments with the comparison fragments so as to obtain a number y of the comparison fragments which includes at least one of the divided fragments. The reference database 320 is constructed by the analyzing-processing module 310 analyzed after cleaving a plurality of reference biological samples by the sequencing platform 200 and the reference database 320 includes a male area (the area with a value of the vertical axis greater than the default threshold 2 in Fig. 2), a female area (the area with the value of the vertical axis smaller than the default threshold 1 in Fig. 2), and an abnormal area (the area with the value of the vertical axis between the default threshold 1 and the default threshold 2 in Fig. 2). The comparing module 330 is for comparing the number y with the reference database 320 to determine the number y falling within the abnormal area, the male area, or the female area.

Therefore, by the analyzing-processing module 310, the reference database 320 and the comparing module 330 of the non-transitory machine 300, the known Y chromosome of the human body can be cleaved into a plurality of comparison fragments and can be compared with the divided fragments so as to obtain the number y of the comparison fragments which includes at least one of the divided fragments and can efficiently confirm whether the sexuality of the fetus is abnormal or not. The details of the non-invasive fetal sex determination system 100 will describe in the following descriptions.

The DNA of the tested sample T1 and the reference biological samples of the non-invasive fetal sex determination system 100 are obtained from the *in vitro* biological samples of the pregnant woman, such as the blood and the urine of the pregnant woman. Both the DNA of the tested sample T1 and the reference biological samples contain the nucleic acids of the pregnant woman and the nucleic acids of the fetus, and the sexuality of the reference biological samples of the fetus are confirmed. In other words, the reference database 320 is constructed based on the same sexuality in the actual sexuality and the detected sexuality of the reference biological samples.

The sequencing platform 200 is a sequencing instrument, wherein the sequencing instrument is a conventional technique so that the details of the sequencing instrument dose not describe here. After obtaining the DNA of the tested sample T1, the sequencing platform 200 cleaves the DNA of the tested sample T1 into a plurality of divided fragments. In this embodiment, the sequencing platform 200 can process whole genome sequencing and can set up the output length of the sequencing DNA about 300 base pairs. Because the sequencing platform 200 can delete the DNA with weak signal automatically, the lengths of the divided fragments can range from 10 base pairs to 300 base pairs.

The non-transitory machine 300 can be flexible discs, optical discs, compact discs (CDs), digital video discs (DVDs), magneto-optical discs, read-only memories (ROMs), random-access memories (RAMs), erasable programmable read only memories (EPROMs), electrically erasable programmable read-only memories (EEPROMs), magnetic card, flash card, or readable devices of any types of media/machines which is suitable for storing instructs, but not limited thereto.

The analyzing-processing module 310 of the non-transitory machine 300 cleaves the known Y chromosome sequence of the human body at the length of 50 kilo base pairs, the length of 100 kilo base pairs, and the length of 200 kilo base pairs into a plurality of comparison fragments, and then compares the divided fragments with the comparison fragments so as to obtain the number y of the DNA of the tested sample T1. For example, a read number is 1E-07 (a scientific symbol) divided fragments, and the Y chromosome sequence is cleaved at the length of 100 kilo base pairs and then produced 594 comparison fragments. In the first comparison, the first comparison fragment is compared with all the divided fragments one by one. The count is 1 if the first comparison fragment includes at least one of the divided fragments, and the count is 0 if the first comparison fragment does not include any divided fragment. In the second comparison, the second comparison fragment is compared with all the divided fragments one by one. The count is 1 if the second comparison fragment includes at least one of the divided fragments, and the count is 0 if the second comparison fragment does not include any divided fragment. After comparing 594 times, the sum of the counts is the number y.

The aforementioned known Y chromosome sequence of the human body is the well-known Y chromosome sequence in this art, and there is no further explanation.

Each of the reference biological samples of reference database 320 is also cleaved into a plurality of divided fragments by the sequencing platform 200, and the divided fragments are also compared with the comparison fragments by the analyzing-processing module 310 so as to obtain a number of the comparison fragments which includes at least one of the divided fragments. In Fig. 2, the vertical axis is a number of the comparison fragments which includes at least one of the divided fragments of each of the reference biological samples, and the horizontal axis is a number of the divided fragments which is cleaved by the sequencing platform 200 of the reference biological samples, and scilicet the number is the read number.

The read number and the number of the reference biological samples which contains the divided fragments with particular sequence are used to plot an x-y scatter plot in EXCEL. As the x-y scatter plot shows, there is a significant difference between the area of male and the area of female so as to construct the male area, the female area, and the abnormal area.

Hence, a linear trend line can be obtained by the scatter plot in EXCEL beforehand. In this embodiment, an equation of the trend line obtained from calculating all of the reference biological samples is y=4E-06x+65, wherein the 4E-06 is the scientific symbol, y is the number of comparison fragments which includes at least one of the divided fragments, and x is the read number. Besides, the plus and minus 10% area of the trend line equation y=4E-06x+65 are set up as the abnormal area. In other words, the minus 10% value of the trend line equation y=4E-06x+65 is the straight line of the default threshold value 1 obtained after calculating, and the plus 10% value of the line of trend equation y=4E-06x+65 is the straight line of the default threshold value 2 obtained after calculating. After the aforementioned calculations, the reference database 320 contains the male area (the area with the value of the vertical axis greater than the default threshold 2), the female area (the area with the value of the vertical axis smaller than the default threshold 1), and the abnormal area (the area with the value of the vertical axis between the default threshold 1 and default threshold 2).

The person having ordinary skill in the art knows that when the length of the out-put DNA sequence of the sequencing platform 200 changes, the particular sequence changes, or the reference biological samples change, the equation of the aforementioned trend line will change. In spite of that, the male area, female area and abnormal area still can be distinguished by setting up the plus and minus 10% areas of the recalculated equation of the trend line as the abnormal area. Although the aforementioned example is using the EXCEL to calculate the trend line, it is also able to calculate the equation of the trend line by using other programs in other embodiments and not be limited thereto.

The comparing module 330 can compared the number y of the DNA of the tested sample T1 with the reference database 320. In other words, the comparing module 330 compares the number y and the read number of the DNA of the tested sample T1 with the reference database 320, and determines whether the sexuality of the DNA of the tested sample T1 is abnormal or not according to a data point of the number y. When the data point of the number y falls within the abnormal area, the DNA of the tested sample T1 is determined as sexual abnormal. When the data point of number y falls within the male area, the DNA of the tested sample T1 is determined as male (male with normal sexuality). When the data point of number y falls within the female area, the DNA of the tested sample T1 is determined as female (female with normal sexuality).

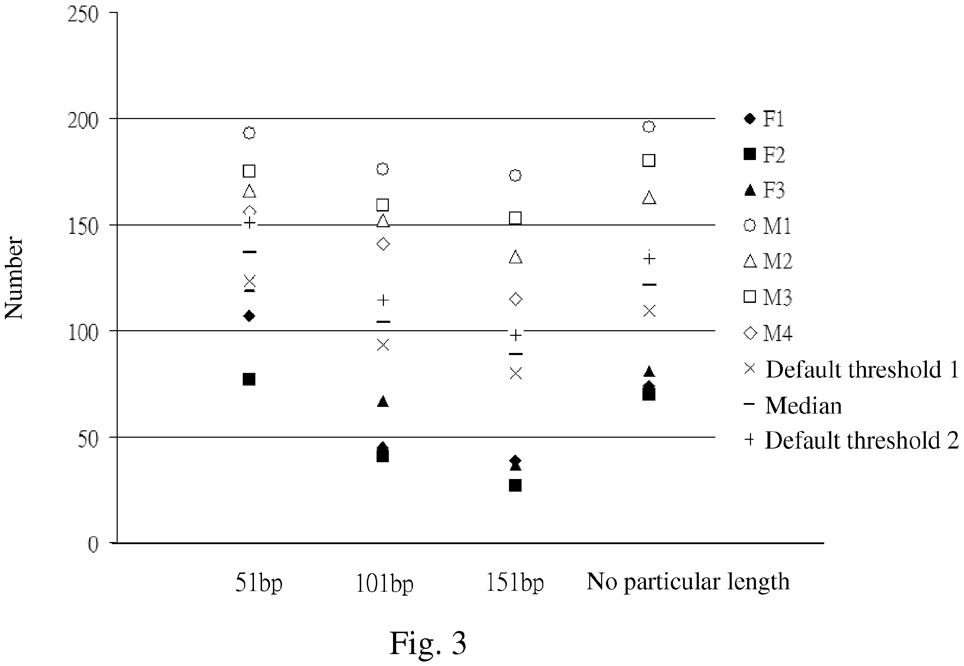

Fig. 3 is another chart of a reference database 320 of the non-invasive fetal sex determination system 100 of Fig. 1. The non-invasive fetal sex determination system 100 also can process the data of the DNA of the tested sample T1.

The analyzing-processing module 310 of non-transitory machine 300 can include a processing unit 311 and a mathematical unit 312. The processing unit 311 is for selecting the divided fragments with a length greater than a particular value and cleaving the divided fragments selected based on the particular value into a plurality of analyzed fragments, and the mathematical unit 312 is for calculating the number y of the comparison fragments which includes at least one of the analyzed fragments, wherein the reference biological samples of reference database 320 are processed by the analyzing-processing module 310 beforehand to construct the male area, the female area, and the abnormal area. When the number y falls within the abnormal area, the DNA of the tested sample T1 is determined as sex abnormal.

As the aforementioned descriptions, the length of the divided fragments of the DNA of the tested sample T1 ranges from 10 base pairs to 300 base pairs, so the processing unit 311 can set up the particular value as 51 base pairs, 101 base pairs, or 151 base pairs and select the divided fragments with the length larger than the particular value. Then the processing unit 311 cleaves the divided fragments selected based on the particular value into a plurality of analyzed fragments. In other words, when the particular value is 51 base pairs, the divided fragments with the length larger than 51 base pairs will be selected. For example, 2.5E-06 divided fragments are selected from 5E-06 divided fragments, and then the selected 2.5E-06 divided fragments are cleaved based on the length of 51 base pairs so as to obtain 6E-06 analyzed fragments. The aforementioned number of the divided fragments and the analyzed fragments are one of the examples, and the actual number of the divided fragments and the analyzed fragments depend on the status of the DNA of the tested sample T1.

Owing to the divided fragments of the DNA of the tested sample T1 are length-selected, the reference biological samples of the reference database 320 should be dealt with in the same way so as to construct the male area, the female area, and the abnormal area corresponding to the particular value. For example, there are 7 reference biological samples in Fig. 3, wherein 3 of the 7 reference biological samples are female (F1 to F3), and 4 of the 7 reference biological samples are male (M1 to M4). Each of the reference biological samples is analyzed to obtain a plurality of analyzed fragments. The number of the comparison fragments which includes at least one of the analyzed fragments is set up as the vertical axis, and the horizontal axis is corresponding to the different particular values. The comparison fragments including at least one of the analyzed fragments in the 3 females of the reference biological samples are calculated so as to obtain an average value of the female. The comparison fragments including at least one of the analyzed fragments in the 3 males of the reference biological samples are also calculated so as to obtain an average value of the male. The average value of female and the average value of male are calculated so as to obtain a median, and then the plus 10% area and the minus 10% area of the median are set up as a particular abnormal area. In other words, the default threshold value 1 is obtained after calculating the minus 10% of the median, and the default threshold value 2 is obtained after calculating the plus 10% of the median. The reference database 320 can construct the male area (the area with the value of the vertical axis greater than the default threshold value 2), the female area (the area with the value of the vertical axis lower than the default threshold value 2) and the abnormal area (the area with the value of the vertical axis between the default threshold value 1 and 2) corresponded to the particular value according to the aforementioned calculations.

The comparing module 330 can compare the number y of the DNA of the tested sample T1 with the reference database 320. The DNA of the tested sample T1 is sex abnormal when the number y falls within the abnormal area, the DNA of the tested sample T1 is male when the data point of the number y falls within the male area, and the DNA of the tested sample T1 is female when the data point of the number y falls within the female area.

The non-invasive fetal sex determination system 100 can detect the DNA of the tested sample T1 falling within the abnormal area and then detect the sex abnormality of the fetus, wherein the sex abnormality can be Y chromosome microdeletion. The number y of the DNA of the tested sample T1 with Y chromosome microdeletion is on the low side and is similar with that of the female fetus, wherefore the conventional sexuality determination system may determine the DNA of the tested sample T1 with Y chromosome microdeletion as the female fetus. However, the number y of the DNA of the tested sample T1 with Y chromosome microdeletion will fall within the abnormal area so that the DNA of the tested sample T1 will be processed further analysis and examination in the non-invasive fetal sex determination system 100.

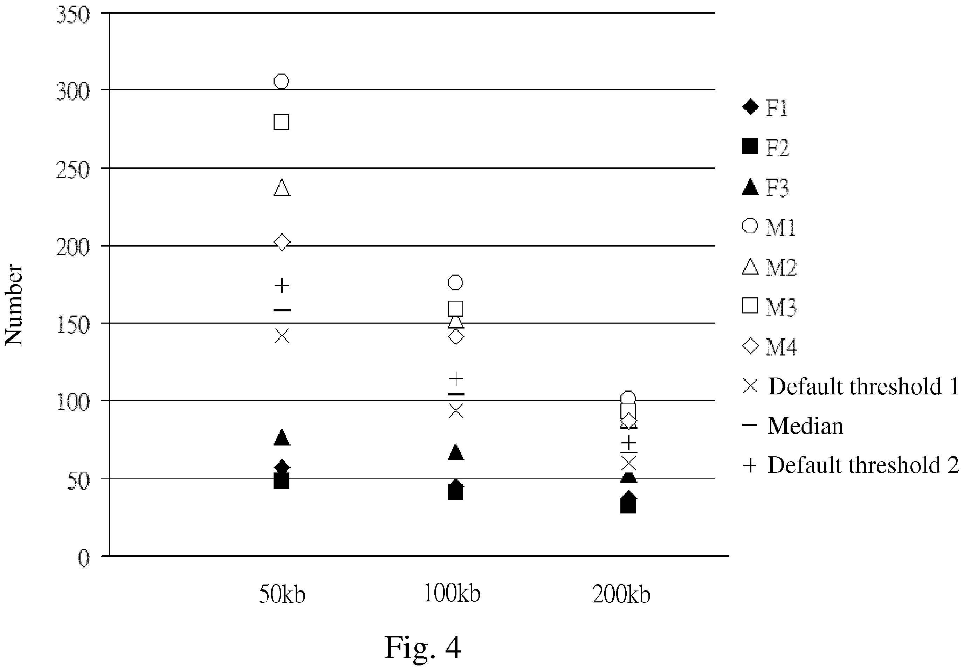

Fig. 4 is still another chart of a reference database 320 of the non-invasive fetal sex determination system 100 of Fig. 1. In Fig. 4, when the Y chromosome sequence of the human body is cleaved at the length of 50 kilo base pairs, the length of 100 kilo base pairs, or the length of 200 kilo base pairs, all of the female area, the male area and the abnormal area can be determined based on the aforementioned methods in Fig. 3.

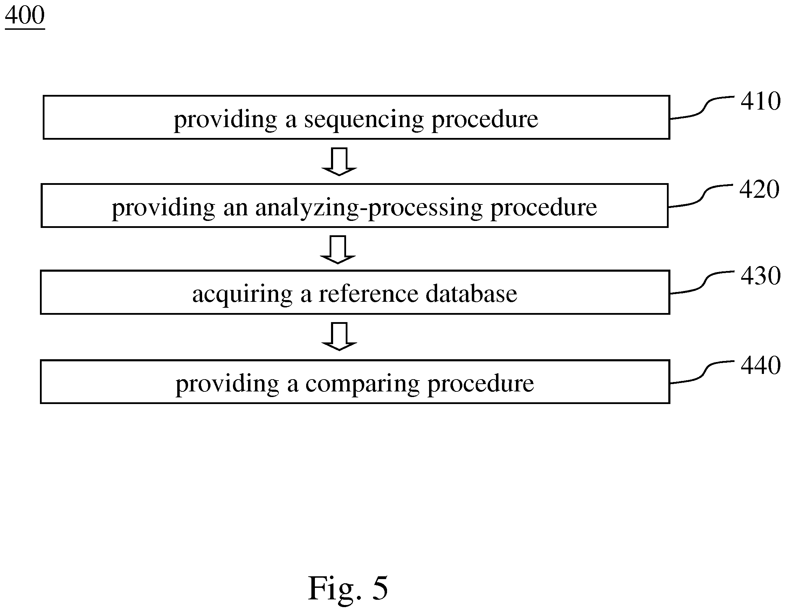

Please refer to Fig. 1, Fig. 2 and Fig. 5 together, wherein the Fig. 5 is a flow chart of a non-invasive fetal sex determination method 400 according to another embodiment of the present disclosure. The non-invasive fetal sex determination method 400 includes step 410, step 420, step 430 and step 440.

The step 410 is providing a sequencing procedure for cleaving a DNA of a tested sample T1 into a plurality of divided fragments by a sequencing platform 200. As the aforementioned descriptions, the divided fragments are with different lengths ranging from 10 base pairs to 300 base pairs after cleaving by the sequencing platform 200.

The step 420 is providing an analyzing-processing procedure for cleaving a known Y chromosome sequence of a human body into a plurality of comparison fragments and comparing the divided fragments with the comparison fragments to obtain a number y of the comparison fragments which includes at least one of the divided fragments, wherein the analyzing-processing module 310 of the non-transitory machine 300 cleaves the Y chromosome sequence into a plurality of comparison fragments and compares the divided fragments with the comparison fragments to obtain the number y of the comparison fragments which includes at least one of the divided fragments so as to obtain primary sexuality analyzed data of the fetus.

The step 430 is acquiring a reference database 320 which is constructed by an analyzing-processing module 310 analyzed after cleaving a plurality of reference biological samples by the sequencing platform 200, wherein the reference database includes a male area, a female area, and an abnormal area. The acquisition and area-divided method of the reference database 320 are as the aforementioned descriptions and not repeat again.

The step 440 is providing a comparing procedure for comparing the number y with the reference database 320. When the number y falls within the abnormal area, the number y can be compared by the comparing module 330 of the non-transitory machine 300 to determine that the DNA of the tested sample T1 is sex abnormal.

In other embodiments, the analyzing-processing procedure of step 420 further includes selecting the divided fragments with a length greater than a particular value, cleaving the divided fragments selected based on the particular value into a plurality of analyzed fragments, and calculating the number of the comparison fragments which include at least one of the analyzed fragments, wherein the reference biological samples of the reference database 320 are processed beforehand to construct the male area, the female area, and the abnormal area corresponding to the particular value. The processing unit 311 selects the divided fragments that the length is greater than the particular value, wherein the particular value is 51 base pairs, 101 base pairs, or 151 base pairs. The mathematical unit 312 calculates the number y of the comparison fragments which includes a particular sequence. The details are described in the aforementioned embodiments and not repeat again here.

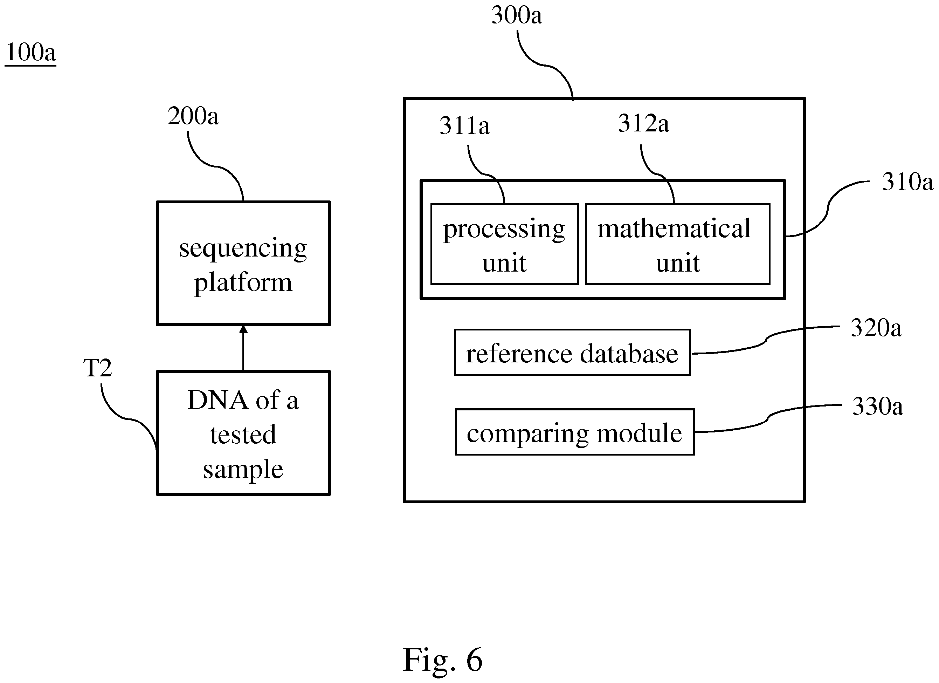

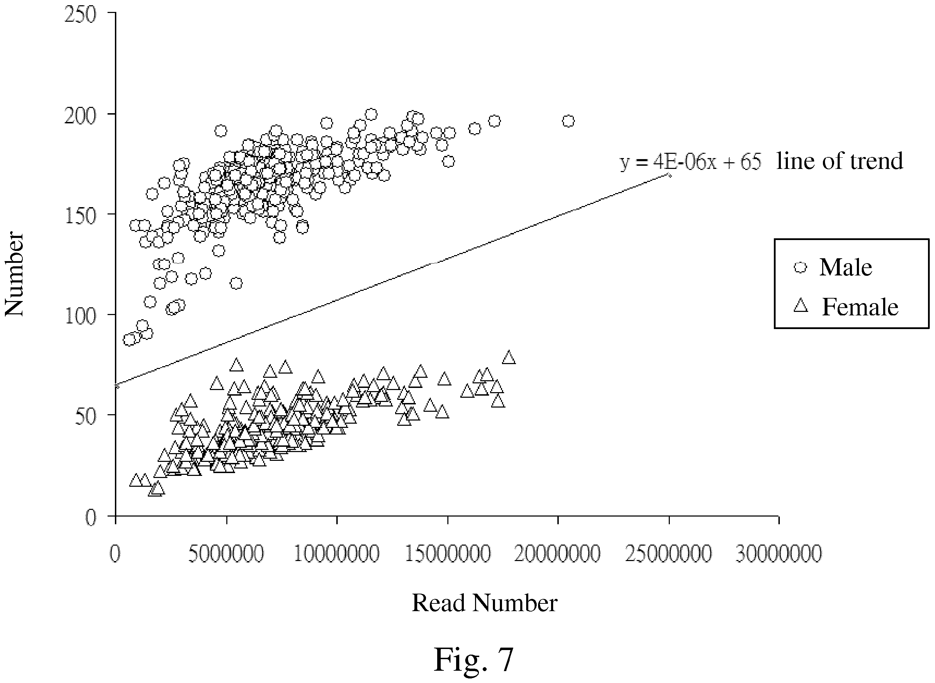

Please refer to Fig. 6 and Fig. 7 together, wherein the Fig. 6 is a block diagram of a non-invasive fetal sex determination system 100a according to still another embodiment of the present disclosure, and the Fig. 7 is a chart of a reference database 320a of the non-invasive fetal sex determination system 100a of Fig. 6. The non-invasive fetal sex determination system 100a includes a sequencing platform 200a and a non-transitory machine 300a.

The sequencing platform 200a is for cleaving a DNA of a tested sample T2 into a plurality of divided fragments, the non-transitory machine 300a is for storing a program used to detect the sexuality of the DNA of the tested sample T2. The non-transitory machine 300a includes an analyzing-processing module 310a, a reference database 320a and a comparing module 330a. The analyzing-processing module 310a is for cleaving a known Y chromosome sequence of a human body into a plurality of comparison fragments and comparing the divided fragments with the comparison fragments to obtain a number y of the comparison fragments which includes at least one of the divided fragments. The reference database 320a is constructed by the analyzing-processing module 310a analyzed after cleaving a plurality of reference biological samples by the sequencing platform 200a, wherein the reference database includes a male area (the area with a value of the vertical axis greater than a trend line of Fig. 7) and a female area (the area with the value of the vertical axis smaller than the trend line of Fig. 7). The comparing module 330a is for comparing the number y with the reference database 320a and determining that the DNA of the tested sample T2 is male when the number y falls within the male area, and the DNA of the tested sample T2 is female when the number y falls within the female area.

By the analyzing-processing module 310, reference database 320 and comparing module 330 of the non-transitory machine 300, the number y of the comparison fragments which includes at least one of the divided fragments is obtained and can efficiently confirm the sexuality of the fetus.

The sequencing platform 200a, the non-transitory machine 300a and the analyzing-processing module 310 of the non-invasive fetal sex determination system 100a are similar to the sequencing platform 200, the non-transitory machine 300 and analyzing-processing module 310 in Fig. 1, but the reference database 320a only includes the trend line without the default threshold value 1 and the default threshold value 2, wherein the calculated methods of the trend line is calculated in the same calculated method of the trend line in Fig. 2. Thus, the reference database 320a includes the male area and the female area based on the trend line.

The comparing module 330a can compare the number y of the DNA of the tested sample T2 with the reference database 320a and determine the sexuality of the DNA of the tested sample T2 based on the data point of the number y.

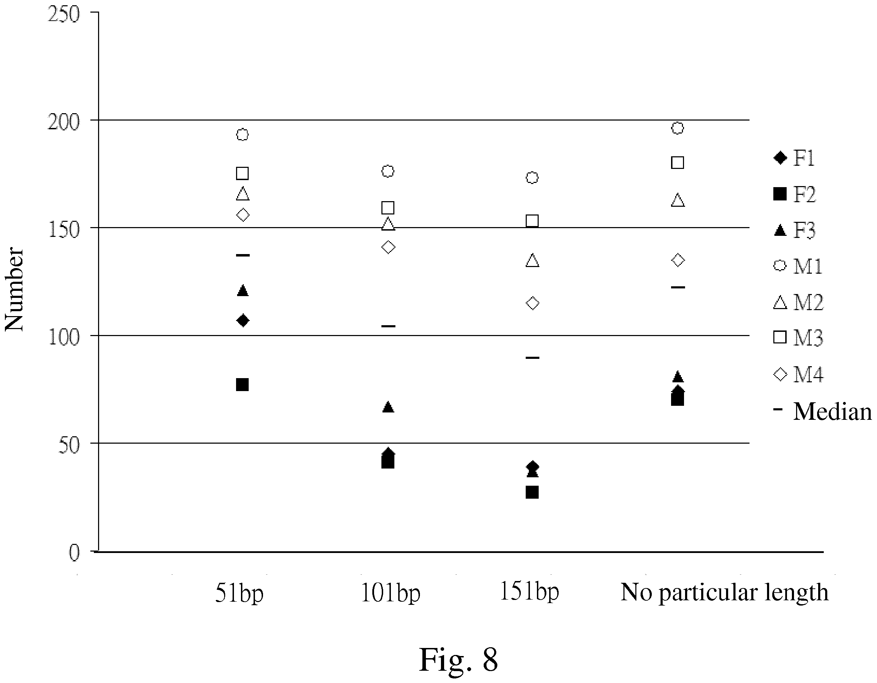

Please refer to Fig. 8 and Fig. 6 together, wherein the Fig. 8 is another chart of a reference database 320a of the non-invasive fetal sex determination system 100a of Fig. 6. In this embodiment, the analyzing-processing module 310a also includes a processing unit 311 a and a mathematical unit 312a, which are similar to the processing unit 311 and mathematical unit 312 in Fig. 1, but the reference database 320a only includes the median without the default threshold value 1 and the default threshold value 2, wherein the median is calculated in the same calculated method in Fig. 3. In other embodiments, the dividing value between the male area and the female area can be constructed by different calculated methods. For example, when the particular value is the 101 base pairs, the dividing value of the male area and the female area can be set up at 90. The reference database 320a can be constructed by the male areas and the female areas corresponding to different particular values and can be compared by the comparing module 330a based on different particular values.

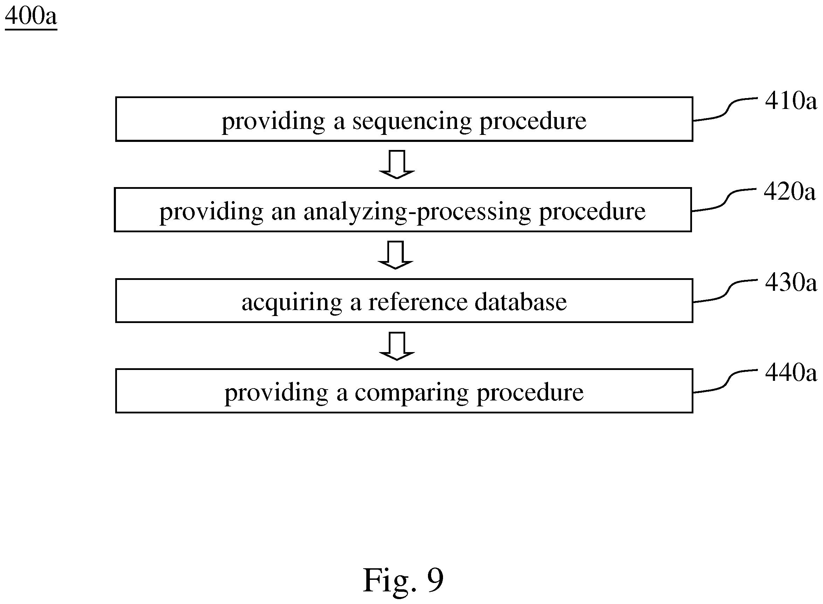

Please refer to Fig. 9 and Fig. 6 together, wherein the Fig. 9 is a flow chart of a non-invasive fetal sex determination method 400a according to yet another embodiment of the present disclosure.

The non-invasive fetal sex determination method 400a includes step 410a, step 420a, step 430a and step 440a, wherein the step 410a and step 420a are similar with the step 410 and step 420 in Fig. 5.

The step 430a is acquiring a reference database 320a which is constructed by an analyzing-processing module 310a analyzed after cleaving a plurality of reference biological samples by the sequencing platform 200a, wherein the reference database 320a includes a male area and a female area. The acquiring method and the area-divided method of the reference database 320a are identical to the aforementioned descriptions and not repeat again here.

The step 440a is providing a comparing procedure for comparing the number y with the reference database 320a. The DNA of the tested sample T2 is male when the number y falls within the male area, and the DNA of the tested sample T2 is female when the number y falls within the female area.

In other embodiments, the analyzing-processing procedure of step 420a further includes selecting the divided fragments with a length greater than a particular value and cleaving the divided fragments selected based on the particular value into a plurality of analyzed fragments, and calculating the number of the comparison fragments which includes at least one of the analyzed fragments. The reference biological samples of the reference database 320a are processed by the analyzing-processing module 310 beforehand to construct the male area and the female area corresponds to the particular value. The processing unit 311 a selects the divided fragments with a length greater than the particular value, wherein the particular value is 51 base pairs, 101 base pairs, or 151 base pairs. The details of the mathematical unit 312 which is used for calculating the number y of the comparison fragments which includes at least one of the analyzed fragments are described in the aforementioned embodiments and not repeat again here.

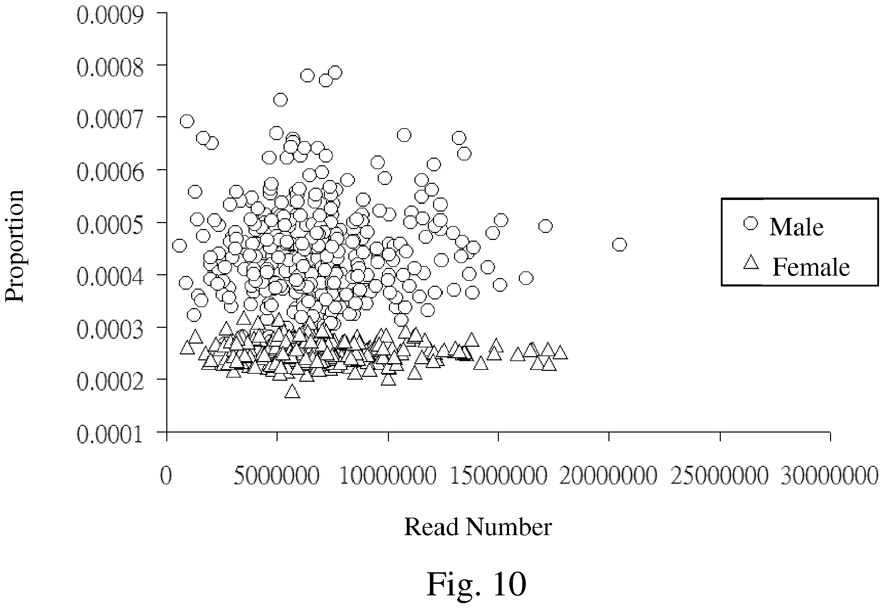

Fig. 10 is a result chart of a conventional non-invasive fetal sex determination system. The conventional non-invasive fetal sex determination method is cleaving the DNA of the tested sample or the chromosome of the reference biological samples into a plurality of fragments and then comparing the fragments directly with a known Y chromosome of the human body. The count is 1 if one of the fragments matches to the known Y chromosome of the human body (the known Y chromosome of the human body includes a sequence of the fragment), and the count is 0 if one of the fragments doesn't match to the known Y chromosome of the human body, thereby calculating a sum of the counts of all fragments. In Fig. 10, the vertical axis is the results of the sum of the counts divided by the sum of the fragments, and the horizontal axis is the read number. The sexuality of the fetus is marked after the baby is born. In the conventional non-invasive fetal sex determination system, a part of the male area and a part of the female area are overlapped and thus the conventional non-invasive fetal sex determination system not only cannot clearly determine the sexuality of the fetus but also cannot confirm whether the sexuality of the fetus is abnormal or not. In Fig. 7, the results of the present disclosure of the non-invasive fetal sex determination method 400a shows that there is a clear distinction between the male area and the female area, hence the non-invasive fetal sex determination method 400a can determine the sexuality of the fetus efficiently and can further confirm the sex abnormality of the fetus by the abnormal area.

According to the aforementioned embodiments, the present disclosure has the advantages described bellowing:

First, by cleaving the known Y chromosome of the human body into a plurality of comparison fragments and cleaving the DNA of the tested sample into a plurality of divided fragments, the non-invasive fetal sex system can analyze the number of the comparison fragments which includes at least one of the analyzed fragments, and then compare the DNA of the tested sample with the male area, female area and abnormal area of the reference database, so as to confirm that whether the sexuality of the DNA of the tested sample is abnormal or not. The DNA of the tested sample is obtained from the specimen of the pregnant woman. Hence it does not worry about the risk of invasive sampling.

Second, by cleaving the known Y chromosome of the human body into a plurality of comparison fragments and cleaving the DNA of the tested sample into a plurality of divided fragments, and analyzing the number of the comparison fragments which includes at least one of the analyzed fragments, the non-invasive fetal sex determination system can compare the DNA of the tested sample with the male area and female area of the reference database, so as to confirm the correct sexuality of the DNA of the tested sample. The determination results of the non-invasive fetal sex determination method of the present disclosure are more accurate than that of the conventional fetal sex determination method.

## Claims

1. A non-invasive fetal sex determination system (100, 100a) comprising:
a sequencing platform (200, 200a) for cleaving a DNA of a tested sample (T1, T2) into a plurality of divided fragments; and
a non-transitory machine readable medium (300, 300a) for storing a program used to detect a sexuality of the DNA of the tested sample (T1, T2), the non-transitory machine readable medium (300, 300a) comprising:
an analyzing-processing module (310, 310a) for cleaving a known Y chromosome sequence of a human body into a plurality of comparison fragments and comparing the divided fragments with the comparison fragments so as to obtain a number of comparison fragments which comprising at least one of the divided fragments;
a reference database (320, 320a) constructed by the analyzing-processing module (300, 310a) analyzed after cleaving a plurality of reference biological samples by the sequencing platform (200, 200a), wherein the reference database (320, 320a) comprises a male area and a female area; and
a comparing module (330, 330a) for comparing the number with the reference database (320, 320a) so as to determine that the DNA of the tested sample (T1, T2) is male when the number falls within the male area and the DNA of the tested sample (T1, T2) is female when the number falls within the female area.

2. The non-invasive fetal sex determination system (100, 100a) of claim 1, wherein a length of the divided fragment ranges from 10 base pairs to 300 base pairs.

3. The non-invasive fetal sex determination system (100, 100a) of claim 2, wherein the analyzing-processing module (310, 310a) comprises:
a processing unit (311, 311 a) for selecting the divided fragments with the length greater than a particular value and cleaving the divided fragments selected based on the particular value into a plurality of analyzed fragments; and
a mathematical unit (312, 312a) for calculating the number of the comparison fragments which comprises at least one of the analyzed fragments;
wherein the reference biological samples of the reference database (320, 320a) are processed by the analyzing-processing module (310, 310a) beforehand to construct the male area and the female area corresponding to the particular value, and the comparing module (330, 330a) determines the DNA of the tested sample (T1, T2) is male when the number falls within the male area and the DNA of the tested sample (T1, T2) is female when the number falls within the female area.

4. The non-invasive fetal sex determination system (100, 100a) of claim 1, wherein the reference database (320, 320a) further comprises an abnormal area and the comparing module (330, 330a) compares the number with the reference database (320, 320a) so as to determine the number falls within the abnormal area, the male area, or the female area.

5. The non-invasive fetal sex determination system (100, 100a) of claim 4, wherein the analyzing-processing module (310, 310a) comprises:
a processing unit (311, 311 a) for selecting the divided fragments with the length greater than a particular value and cleaving the divided fragments selected based on the particular value into a plurality of analyzed fragments; and
a mathematical unit (312, 312a) for calculating the number of the comparison fragments which comprise at least one of the analyzed fragments;
wherein the reference biological samples of the reference database (320, 320a) are processed by the analyzing-processing module (310, 310a) beforehand to construct the male area, the female area, and the abnormal area corresponding to the particular value.

6. The non-invasive fetal sex determination system (100, 100a) of claim 3 or 5, wherein the Y chromosome sequence of the human body is cleaved at the length of 50 kilo base pairs, the length of 100 kilo base pairs, or the length of 200 kilo base pairs.

7. The non-invasive fetal sex determination system (100, 100a) of claim 3 or 5, wherein the particular value is 51 base pairs, 101 base pairs, or 151 base pairs.

8. A non-invasive fetal sex determination method (400, 400a) comprising:
providing a sequencing procedure (410, 410a) for cleaving a DNA of a tested sample (T1, T2) into a plurality of divided fragments by a sequencing platform (200, 200a);
providing an analyzing-processing procedure (420, 420a) for cleaving a known Y chromosome sequence of a human body into a plurality of comparison fragments and comparing the divided fragments with the comparison fragments so as to obtain a number of the comparison fragments which comprising at least one of the divided fragments;
acquiring a reference database (320, 320a) constructed by an analyzing-processing module (310, 310a) analyzed after cleaving a plurality of reference biological samples by the sequencing platform (200, 200a), wherein the reference database (320, 320a) comprises a male area and a female area; and
providing a comparing procedure (440, 440a) for comparing the number with the reference database (320, 320a) so as to determine that the DNA of the tested sample (T1, T2) is male when the number falls within the male area and the DNA of the tested sample is female when the number falls within the female area.

9. The non-invasive fetal sex determination method (400, 400a) of claim 8, wherein a length of the divided fragment ranges from 10 base pairs to 300 base pairs.

10. The non-invasive fetal sex determination method (400, 400a) of claim 8, wherein the analyzing-processing procedure (420, 420a) comprises:
selecting the divided fragments with a length greater than a particular value and cleaving the divided fragments selected based on the particular value into a plurality of analyzed fragments; and
calculating the number of the comparison fragments which comprises at least one of the analyzed fragments;
wherein the reference biological samples of the reference database (320, 320a) are processed by the analyzing-processing module (420, 420a) beforehand to construct the male area and the female area corresponds to the particular value, and the comparing module determines the DNA of the tested sample (T1, T2) is male when the number falls within the male area and the DNA of the tested sample (T1, T2) is female when the number falls within the female area.

11. The non-invasive fetal sex determination method (400, 400a) of claim 8, wherein the reference database (320, 320a) further comprises an abnormal area and the comparing procedure (440, 440a) is for comparing the number with the reference database (320, 320a) to determine the number falling within the abnormal area, the male area, or the female area.

12. The non-invasive fetal sex determination method (400, 400a) of claim 11, wherein a length of the divided fragment ranges from 10 base pairs to 300 base pairs in the sequencing procedure.

13. The non-invasive fetal sex determination method (400, 400a) of claim 12, wherein the analyzing-processing procedure (420, 420a) comprises:
selecting the divided fragments with a length greater than a particular value and cleaving the divided fragments selected based on the particular value into a plurality of analyzed fragments; and
calculating the number of the comparison fragments which comprises at least one of the analyzed fragments;
wherein the reference biological samples of the reference database (320, 320a) are processed by the analyzing-processing module (420, 420a) beforehand to construct the male area, the female area, and the abnormal area corresponding to the particular value.

14. The non-invasive fetal sex determination method (400, 400a) of claim 10 or 13, wherein the Y chromosome sequence of the human body is cleaved at the length of 50 kilo base pairs, the length of 100 kilo base pairs, or the length of 200 kilo base pairs.

15. The non-invasive fetal sex determination method (400, 400a) of claim 10 or 13, wherein the particular value is 51 base pairs, 101 base pairs, or 151 base pairs.
